# EUROPEAN PATENT APPLICATION

(11) **EP 4 335 470 A1**
(43) Date of publication of application: **13.03.2024**
(21) Application number: 22194980.3
(22) Date of filing: 09.09.2022
(51) Int. Cl.: A61M 5/142

(54) **DRUG DELIVERY DEVICE**

(71) Applicant: Sensile Medical AG, 4600 Olten (CH)
(72) Inventor: BÜRLI, Fabian, 4655 Stüsslingen (CH); LAGORGETTE, Pascal, 2502 Bienne (CH)
(74) Representative: reuteler & cie SA

(57) **Abstract**

A drug delivery device (1), including a delivery unit (3) comprising a subcutaneous delivery system including a needle support (10), an injection needle (9) mounted on the needle support, and an injection needle actuation mechanism configured to engage and slidably move the needle support from a retracted position where the injection needle is within a housing (2) of the drug delivery device to an extended delivery position where the injection needle projects through a skin contact wall of the housing. The delivery unit further comprises a needle shield mechanism comprising a needle shield cover (16) slidably movable from a retracted position within the housing to an extended position covering the needle and a spring (26) biasing the needle shield cover towards the extended position, the needle support comprising a shoulder engaging a shoulder of the needle shield cover configured to hold the needle shield cover in the retracted position prior to actuation of the injection needle actuation mechanism.

## Description

### TECHNICAL FIELD

This invention relates to a drug delivery device for subcutaneous administration of a liquid drug. The invention in particular relates to a drug delivery device in the form of a patch device.

### DESCRIPTION OF RELATED ART

Drug delivery devices in the form of a patch device for mounting on a patient's skin for subcutaneous delivery of liquid drug are known. It is known to provide drug delivery devices in the form of a patch device with a single use disposable component assembled to a reusable component containing drive and control electronics, or as a single disposable component.

The reliability, safety, compactness and ease of use of drug delivery devices worn by a patient is important. For disposable components, the amount of parts and consequently cost of the disposable device is also an important consideration.

For safety of use of the drug delivery device, it is also important to ensure that it may only be actuated when attached to a patient's skin, that it remains sterile until administration of the drug, and that after use and removal the disposable portion of the device cannot be reused or harm someone.

In known drug delivery devices provided as patch devices that are placed against a patient's skin and held by an adhesive, an injection needle is actuated upon activation of the device for the sub-cutaneous delivery of the liquid medication. After use, the injection needle is retracted into the drug delivery device to avoid injury after removal of the patch device from the patient's skin. In certain patch devices, the needle is used to insert a soft cannula and thus performs an insertion and retraction movement prior to pumping of the liquid medication, and in certain patch devices a hollow rigid needle is inserted and remains in an extended position for sub-cutaneous drug delivery and retracts at the end of the drug delivery prior to removal of the patch device from the patient's skin. In certain drug delivery devices, it is also known to provide a needle guard that covers the needle after use of the drug delivery device. It is generally preferred to retract the needle into the drug delivery housing, however in the case of delivery devices with rigid needles (instead of soft cannulas) which have less complex mechanisms than devices with soft cannulas, a drawback is that the retraction at the end of the drug delivery cycle requires power and if there is a failure of the device or a loss of power it is possible that the needle remains in an extended position. Moreover, in many devices a retraction mechanism requires a somewhat complex arrangement, in particular to ensure that once retracted the needle remains in the retracted position. Systems provided with needle guards are generally quite bulky and conventional needle guards are not adapted for compact patch pump devices.

There is need to reduce the complexity of mechanisms operated automatically in a drug delivery device to increase reliability and decrease costs, and further to increase the compacity of the medical device.

### SUMMARY OF THE INVENTION

In view of the foregoing, it is an object of the invention to provide a drug delivery device, in particular in the form of a patch device, with a disposable unit, or as an entirely disposable device, for administration of a liquid drug that is safe, reliable, and compact.

It is advantageous to provide a drug delivery device that is easy to use.

It is advantageous to provide a drug delivery device that is economical to produce.

Objects of the invention have been achieved by providing the drug delivery device according to claim 1. Dependent claims set forth various advantageous embodiments of the invention.

Disclosed herein is a drug delivery device including a delivery unit comprising a subcutaneous delivery system including a needle support, an injection needle mounted on the needle support, and an injection needle actuation mechanism configured to engage and slidably move the needle support from a retracted position where the injection needle is within a housing of the drug delivery device to an extended delivery position where the injection needle projects through a skin contact wall of the housing.

The delivery unit further comprises a needle shield mechanism comprising a needle shield cover slidably movable from a retracted position within the housing to an extended position covering the needle and a spring biasing the needle shield cover towards the extended position, the needle support comprising a shoulder engaging a shoulder of the needle shield cover configured to hold the needle shield cover in the retracted position prior to actuation of the injection needle actuation mechanism.

In an advantageous embodiment, the needle support comprises a retracted position holder releasably holding the needle support and needle shield mechanism in the retracted position prior to actuation of the injection needle actuation mechanism.

In an advantageous embodiment, the retracted position holder comprises a clasp having at least one elastic arm releasable under an actuation force applied by the injection needle actuation mechanism.

In an advantageous embodiment, the retracted position holder comprises a pin engaged by the clasp in the retracted position, the pin extending from the needle support.

In an advantageous embodiment, the needle shield cover is slidably mounted in a needle shield guide comprising a shield receiving cavity, the needle shield guide integrally formed or rigidly fixed to the housing or a housing part of the drug delivery device.

In an advantageous embodiment, the needle shield mechanism comprises a skin contact wall configured to protrude slightly beyond a skin contact wall of a housing of the drug delivery device during drug delivery.

In an advantageous embodiment, the needle shield mechanism comprises a skin contact wall comprising a needle orifice and upstanding from the skin contact wall sidewalls and a top wall engaging a top shoulder of the needle support.

In an advantageous embodiment, the spring is in the form of a conical coil spring mounted between a top wall of the needle shield cover and a wall or support of a housing component of the delivery unit.

In an advantageous embodiment, the drug delivery device comprises a pumping system comprising a pump engine, the pump engine including
a stator,
a rotor rotatably and axially slidably mounted at least partially in the stator, the rotor comprising a first axial extension having a first diameter and the second axial extension having a second diameter greater than the first diameter,
a first valve formed by a first valve seal mounted on the stator around the first axial extension, in conjunction with a first channel in the rotor that is configured to allow fluidic communication across the first valve seal when the first valve is in an open position, and a second valve formed by a second valve seal mounted on the stator around the second axial extension, in conjunction with a second channel in a rotor that is configured to allow fluidic communication across the second valve seal when the second valve is an open position.

In an advantageous embodiment, the needle actuation mechanism comprises an actuation disc and an actuation lever, the actuation lever comprising a pivot portion and a lever arm extending therefrom configured to engage an indent in the actuation disc upon initial actuation of the drug delivery device.

In an advantageous embodiment, the actuation disc is coupled to the rotor of the pump engine.

In an advantageous embodiment, said pin extending from the needle support further engages the lever arm.

In an advantageous embodiment, the drug delivery device further include a drive unit comprising an electronic control system, a power source, and a pump drive, wherein the pump drive comprises a rotary electrical motor and a coupling interface coupled to an output shaft of the rotary electrical motor, the coupling interface coupling to a drive coupling interface of the pumping system of the delivery unit, the pump drive providing torque to a rotor of the pumping system.

Further objects and advantageous features of the invention will be apparent from the claims, from the detailed description, and annexed drawings, in which:

### BRIEF DESCRIPTION OF THE DRAWINGS

Figure 1a is a perspective view of a drug delivery device according to an embodiment of the invention;
Figure 1b is another perspective view of the drug delivery device according to an embodiment of the invention;
Figure 1c is a perspective view of the drug delivery device of figures 1a and 1b with disposable and re-usable units separated;
Figure 2a is a perspective view of a bottom side of a portion of a delivery unit of the drug delivery device of figures 1a to 1c;
Figure 2b is a view similar to figure 2a with a housing portion removed;
Figure 2c is a view of the components of figure 2b from a back side;
Figure 2d is a view of the device of figure 2b seen from a top side;
Figure 2e is a view in cross section of a pump engine of a pumping system of the delivery unit;
Figure 3a is a plan view of the device of figure 2b with a needle in a retracted position prior to use;
Figure 3b is a view in cross-section through the needle and needle shield mechanism of figure 3a;
Figure 3c is a view of a portion of figure 3a with an actuation lever removed to better show a retracted position holder and clasp of the subcutaneous delivery mechanism;
Figure 4a is a view similar to figure 3a with the needle shown in the extended drug delivery position;
Figure 4b is a view in cross-section through the needle and needle shield mechanism of figure 4a;
Figure 5a is a view similar to figure 4a after removal of the device from a patient showing the needle shield in an extended position;
Figure 5b is a cross-sectional view through the needle and needle shield mechanism of figure 5a;
Figure 6a is a perspective view showing the needle in the extended position during drug delivery with the needle shield mechanism retracted;
Figure 6b is a detail view in cross-section of the device of figure 6a showing a portion of the needle shield mechanism with a locking latch;
Figure 7a is a perspective view similar to figure 6a showing the needle shield in the extended position after drug delivery and removal of the patch device from the patient;
Figure 7b is a view similar to figure 6b showing the locking latch engaging a locking shoulder on the needle shield when the shield is in the extended position of figure 7a.

### DETAILED DESCRIPTION OF EXEMPLARY EMBODIMENTS

Referring to the figures, a drug delivery device 1 according to embodiments of the invention comprise a housing 2, a delivery unit 3, and a control or drive unit 4, the delivery unit 3 and the control or drive unit 4 being assembled within the housing 2. The housing 2 may be made of two or more parts allowing assembly of the delivery unit, drive unit and any other components within the housing.

In the illustrated embodiments, the drug delivery device for subcutaneous administration of a liquid drug (medicament) comprises a disposable portion formed by the delivery unit that may be assembled to a reusable portion formed by the drive unit, which includes electronics and a power supply. The delivery unit 3 is mounted in a first housing portion of the drug delivery device and the drive unit 4 in a separable second housing portion such that the drive unit 4 can be reused with subsequent delivery units. An example of a drug delivery device with single use disposable and reusable parts is described for instance in WO 2020109409.

Although the embodiments shown in the figures concern a two-part drug delivery device with disposable and reusable units, within the scope of the invention for various aspects described herein, the drug delivery device may be a single use disposable unit. The administration may occur in a single dose over a short period of time, typically less than 1 hour, for instance around 30 minutes or less. A single use disposable drug delivery device may also be used for subcutaneous injection of a liquid drug over an extended period of time from a few hours to a few days. Depending on the volume of the drug to be injected, the drug delivery device may also be configured to inject the liquid drug within a few minutes.

The drug delivery device includes a user interface 46 that may include one or more buttons for actuating the drug delivery device, light and/or sound status indicators, and optionally a screen or other display for presenting information to an operator of the device.

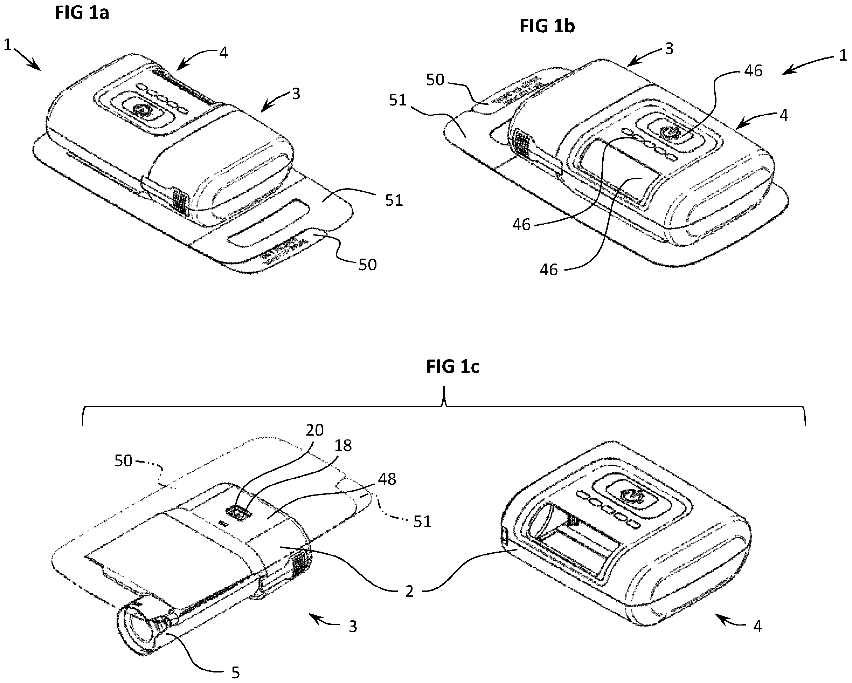

Drug delivery devices according to embodiments of the invention may advantageously be configured as a patch device for mounting on a patient's skin. Referring to figures 1a to 1c, an adhesive layer 51 may be provided on an outer surface of a skin contact wall 48 of the housing 2 covered by a protective film 50 that may be peeled off the adhesive layer 51 prior to placing the adhesive layer on the patient's skin at the site of injection. A needle orifice 20 on the skin contact side is covered by the protective film 50 prior to use, and allows a transcutaneous injection needle 9 to extend therethrough and pierce the patent's skin upon activation of the drug delivery device 1.

The delivery unit 3 comprises a drug container 5, for instance a drug cartridge, containing a liquid drug and a liquid flow system for channeling the liquid drug to a patient subcutaneously.

The drug delivery device further includes a pumping system that causes liquid from the drug container to be pumped to the injection needle 9 once the drug delivery device has been activated.

In an embodiment, the delivery unit 3 incorporates the pumping system 8 that causes liquid from the container to be pumped to the injection needle 9 once the drug delivery device has been activated. The pumping system comprises a drive coupling interface 40 that couples to a coupling interface of a pump drive of the drive unit 4. The drive unit thus provides the mechanical power via the coupling 40 to drive the pumping system 8.

The pump engine 38 in the delivery unit 3 may advantageously comprise a design and configuration similar to the pump engine described in WO 2007074363, WO 2015015379, WO2019228895A1, or WO2020069926A1, in which a rotor 31 is mounted within a stator 29 and is rotatably and axially movable within the stator 29 in order to pump a fluid from a fluid inlet to a fluid outlet. As known from the above-mentioned publications, the rotor 31 has a pump shaft with first and second diameters surrounded by seals that open and close a fluid channel between the inlet and outlet as the rotor rotates and axially displaces due to a cam mechanism between the stator 29 and rotor 31, whereby during the opening and closing of the valves between the fluid inlet and pumping chamber, respectively between the pumping chamber and outlet, a pumping action is performed.

An important advantage of using such pump engine in embodiments of the present drug delivery device is that there is no direct connection of fluid between the inlet and outlet at any position of the rotor and no actuation of any valves are required, such that a particularly reliable pumping of liquid without leakage is ensured in an easy to operate arrangement. The pump engine is very compact and can be driven directly by a rotary electrical motor of the pump drive in the drive unit 4 without gearing. In effect, because of the differential pumping volume displacement that is defined by the axial displacement of the rotor and the difference between the first and second diameters of the pump shaft, the pumping volume displacement rotation can be easily configured for optimal operation with an electrical motor of a given type rotating with a constant speed. Moreover, the pump engine parts can be made entirely of polymer materials and the rotor may be easily coupled to the pump drive ensuring a sterile barrier between the fluidic portion of the pump engine and the coupling interface.

In other embodiments, the pumping system may be mounted in the drive unit and apply pressure on a plunger of the drug container to push liquid out of the drug container into the subcutaneous delivery system by positive pressure. Various *per se* known means for applying pressure on a drug container plunger may be employed.

In another specific embodiment, the pumping system may comprise a pneumatic pumping system as described in patent application PCT/EP2022/055835.

The pumping system may also include other *per se* well known pumping systems, for instance the motor may drive a linear nut and screw system that presses on the plunger.

The drive unit 4 is configured principally to drive the pumping system, but may also have additional functions such as processing sensing signals, and transmitting and receiving data from an external device via a wireless communication link, for instance using Bluetooth.

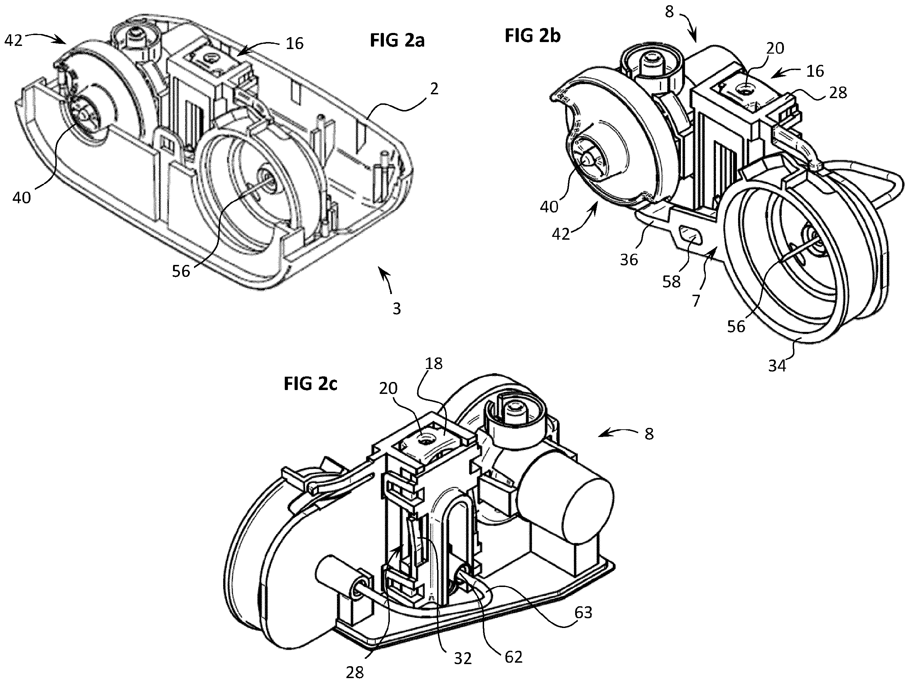

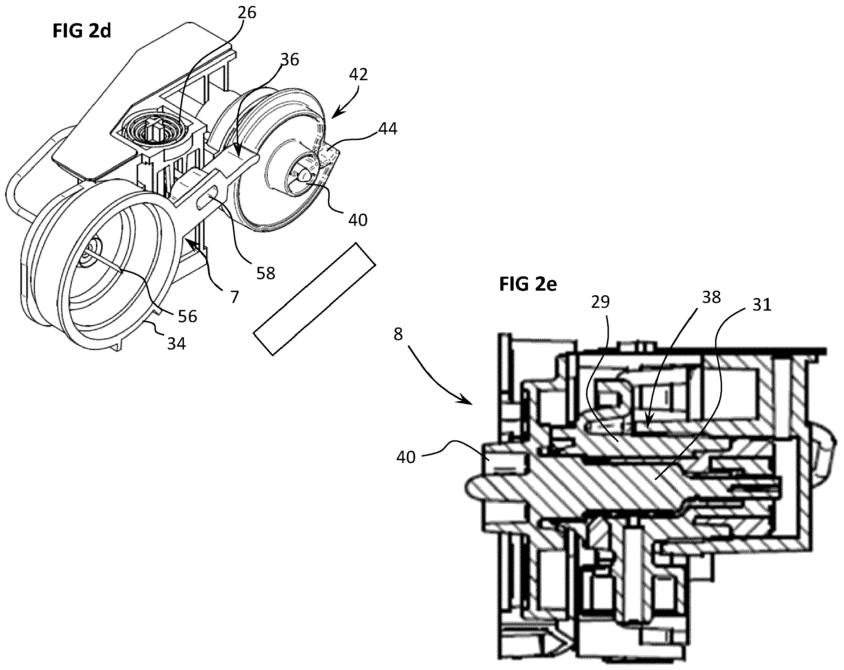

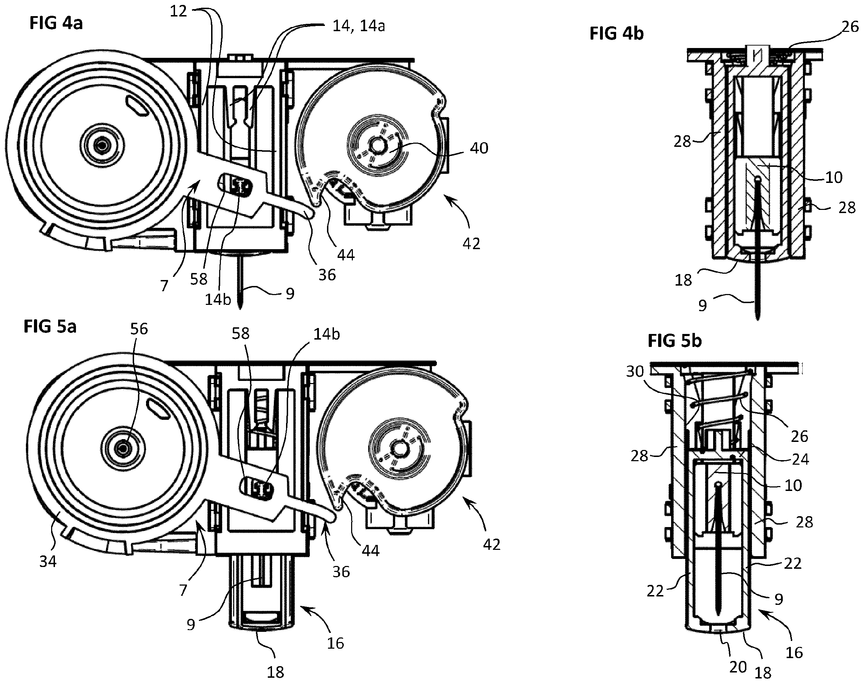

The subcutaneous delivery system comprises a slidable needle support 10 which is slidably mounted within the housing 2, the needle 9 being mounted on the needle support 10 and movable with the needle support 10 from a fully retracted position within the housing 2 of the delivery unit 3 as illustrated in figures 2a to 3b, to a fully extended position during drug administration as illustrated in figure 4a.

The fluidic channel 62 within needle support (see figure 2c) is connected fluidically to the fluid channel in the needle and to a conduit 63 that may advantageously be in the form of a supple tube allowing the sliding movement of the needle support from the retracted to the extended positions. The tube 63 is connected fluidically at its other end to a drug container, for instance to the drug container 5 in the form of a drug cartridge via a septum needle 56 that pierces through the septum of the drug container 5.

In variants, other fluidic connections and drug containers may be provided, for instance the tube may be connected to a drug container of another configuration, for instance a supple container, or a container directly formed in the drug delivery device (instead of being provided as a separate cartridge).

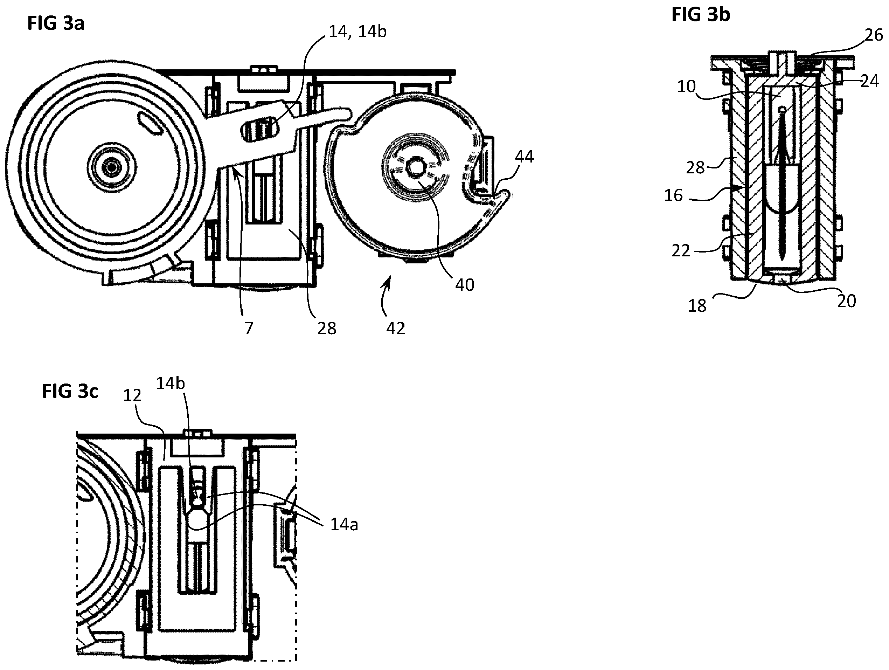

In the present invention, the subcutaneous delivery system may comprise a configuration similar to the delivery system described in WO 2015015379 which is incorporated herein by reference. In such configuration, the rotor 31 of the pump engine 38 comprises an actuation disc 42 comprising an indent 44 that engages the tip of a lever arm 36 connected to a pivot portion 34 of an actuation lever 7. In an embodiment, the pivot portion is in the form of a support ring mounted around a shroud that receives a cap end of the drug container 5. As best seen in reference to figures 2b and 3b, the support ring 34 of the actuation lever 7 is rotatably mounted around a shroud forming a cavity receiving a cap of the container 5, whereas the lever arm 36 extends up to a tip that is configured to catch in the indent 44 of the actuation disc 42 when it is rotated by the pump drive of the drive unit 4.

It may be noted however that in other embodiments, the actuation disc may be independent of the pumping system (i.e. not part of a pump engine) and serve only as part of a needle actuation mechanism. The motor to drive the actuation disc may be a motor of the drive unit that is dedicated for the needle actuation mechanism, or may be a motor that serves both for the needle actuation mechanism and also to drive a pumping system of the drug delivery device that forces the liquid to flow from the drug container to the needle.

As can be seen in figures 2a to 3b, an initial position before first use of the drug delivery device is shown where the tip of the lever arm 65 rests against the outer peripheral surface of the actuation disc 42. When the pumping operation is started upon actuation of the drug delivery device, the actuation disc 42 turns (in the illustration in an anti-clock wise direction) such that the tip of the lever arm 36 engages in an indent 44. Subsequently the continued rotation of the rotor causes the actuation lever 7 to pivot (in the illustration in a clockwise manner) until a fully actuated position shown in figure 4a where the injection needle 9 is fully extended. The actuation lever 7 comprises a slot 58 (or pin) that engages a complementary pin 14b (or slot) on the needle support 10 so as to push the needle support down when the actuation lever is rotated to the needle extended position.

Within the scope of the invention, it would however be possible to have the actuation disk directly engage the slidable needle support without the presence of the actuation lever. For instance, the actuation disk could have an arm or other form of extension that has a slot or pin engaging a complementary pin or slot on the slidable needle support.

According to an aspect of the invention, the delivery unit 3 further comprises a needle shield mechanism comprising a needle shield cover 16 slidably mounted within a needle shield guide 28 of the housing, the needle shield cover 16 slidably movable from a retracted position prior to use of the drug delivery device and during drug delivery when the device is placed against a patient's skin, to an extended position covering the needle as illustrated in figures 5a and 5b after removal of the drug delivery device from the patient's skin.

The needle shield mechanism comprises a spring 26 configured to apply a spring biasing force to push the needle shield cover 16 towards its extended position. In the retracted position, as illustrated in figures 2a to 3b, the spring 26 is in a pre-stressed condition applying a biasing force on the needle shield cover 16.

The needle shield cover 16 is held in the retracted position by the needle support 10 that is positioned within the needle shield cover 16 and a top wall 24 of the needle shield cover presses against a top end of the needle support 10. Although reference is made herein to a top wall 24 and a top end of needle support 10, other elements engaging between the needle shield cover 16 and the needle support 10 may be provided such that the function of holding the needle shield cover in its retracted position by the needle support may be accomplished.

Once the needle is in the extended position for subcutaneous delivery, snapping features may engage into the needle support 10 configured to pull and maintain it in the fully extended position thus pulling indirectly the actuation lever 7 into its lowest position and preventing that it touches the actuation disc 42 in the subsequent pumping revolutions. This may for instance serve to prevent noise that could occur if the tip of the actuation lever 7 touches the actuation disc while the actuation disc rotates.

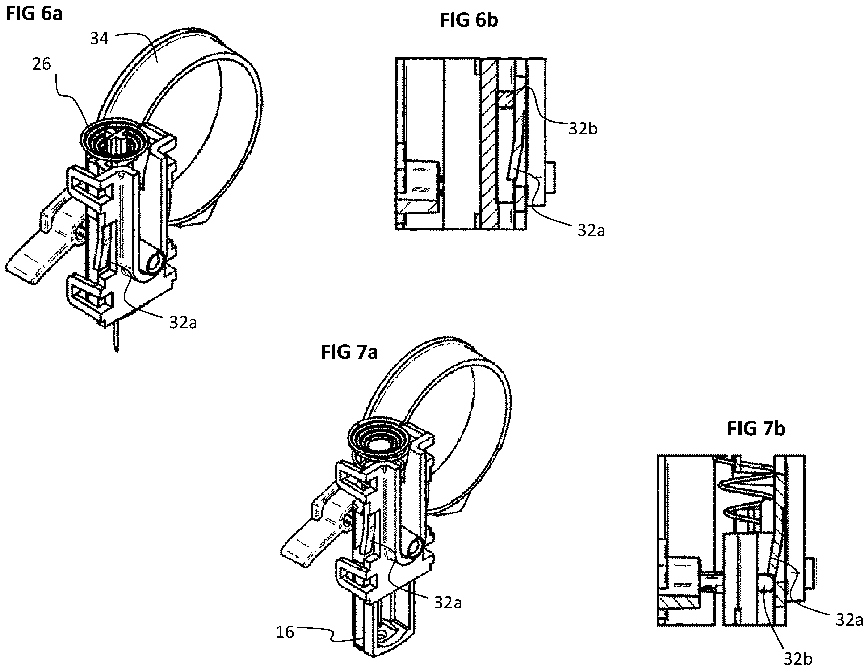

Upon actuation of the drug delivery device and subcutaneous insertion of the needle 9 in the patient's skin, the needle shield cover 16 is no longer held in the retracted position by the needle support 10, however the lower skin contact wall 18 of the needle shield cover 16 presses against the patient's skin and thus prevents extension of the needle shield cover into the extended position. Upon removal of the drug delivery device from the patient, the force of the spring 26 pushes the needle shield cover 16 into the extended position shown in figures 5a, 5b and 7a, 7b as the drug delivery device is being removed from the patient. A locking latch 32a on the needle shield guide 28 engages a complementary locking shoulder 32b on the needle shield cover thus preventing it from being pushed back into the retracted position.

Advantageously, using the needle support to hold the needle shield cover in the retracted position prior to activation of the drug delivery device allows to provide a very compact and simple and reliable needle shield mechanism that deploys automatically after removal of the drug delivery device from the patient's skin, without requiring any power or special motor operations of the drug delivery device.

The needle support 10 and complementary housing may be provided with a retracted position holder mechanism 14, for instance comprising a clasp 14a that engages a pin 14b and which is releasable under a certain biasing force, in particular when the actuation lever is rotated from the retracted to the extended position. The clasp as shown for instance in figure 4a or 3c may simply be formed by elastic arms with protrusions formed in the needle support housing slide 12 and engaging the pin 14b on the needle support 10 that may also serve as the pin engaging the actuation lever 7. The downward force applied by the rotating lever arm on the pin 14b spreads apart the elastic arms of the clasp as the needle support 10 is pulled down towards the extended position.

### List of features

Drug delivery device 1
**Housing 2**
   Skin contact wall 48
      Needle shield orifice
   Adhesive layer 51
   Protective film 50
**Delivery unit 3**
   **Drug container 5**
   Subcutaneous delivery system
      Injection needle 9
      Needle support (slidable) 10
         Fluidic channel 62
      Housing slide 12
      Conduit 63
   Septum needle 56
   Needle shield mechanism
      Needle shield cover 16
         Skin contact wall 18
            Needle orifice 20
         Side walls 22
         Top wall 24
      Spring 26
      Needle shield guide 28
         Shield receiving cavity 30
         Locking members 32a, 32b
   Needle actuation mechanism
      **Actuation disc 42**
         Indent 44
      Actuation lever 7
         Pivot portion 34
            Support ring
         Lever arm 36
            Slot 58
      Retracted position holder 14
         Clasp 14a
         Pin 14b
   **Pumping system 8**
      Pump engine 38
      Stator 29
      Rotor 31
         Drive coupling interface 40
**Drive unit 4**
   Electronic control system
   Power source (battery)
   Pump drive
      Motor
      Coupling interface
   User interface 46

## Claims

1. A drug delivery device (1), including a delivery unit (3) comprising a subcutaneous delivery system including a needle support (10), an injection needle (9) mounted on the needle support, and an injection needle actuation mechanism configured to engage and slidably move the needle support from a retracted position where the injection needle is within a housing (2) of the drug delivery device to an extended delivery position where the injection needle projects through a skin contact wall of the housing, **characterized in that** the delivery unit further comprises a needle shield mechanism comprising a needle shield cover (16) slidably movable from a retracted position within the housing to an extended position covering the needle and a spring (26) biasing the needle shield cover towards the extended position, the needle support comprising a shoulder engaging a shoulder of the needle shield cover configured to hold the needle shield cover in the retracted position prior to actuation of the injection needle actuation mechanism.

2. The drug delivery device according to the preceding claim wherein the needle support comprises a retracted position holder (14) releasably holding the needle support and needle shield mechanism in the retracted position prior to actuation of the injection needle actuation mechanism.

3. The drug delivery device according to the preceding claim wherein the retracted position holder comprises a clasp (14a) having at least one elastic arm releasable under an actuation force applied by the injection needle actuation mechanism.

4. The drug delivery device according to the preceding claim wherein the retracted position holder comprises a pin (14b) engaged by the clasp (14a) in the retracted position, the pin extending from the needle support (10).

5. The drug delivery device according to any preceding claim wherein the needle shield cover is slidably mounted in a needle shield guide (28) comprising a shield receiving cavity (30), the needle shield guide integrally formed or rigidly fixed to the housing (2) or a housing part of the drug delivery device.

6. The drug delivery device according to any preceding claim wherein the needle shield mechanism comprises a skin contact wall (18) configured to protrude slightly beyond a skin contact wall (48) of a housing of the drug delivery device during drug delivery.

7. The drug delivery device according to any preceding claim wherein the needle shield mechanism comprises a skin contact wall (18) comprising a needle orifice (20) and upstanding from the skin contact wall sidewalls (22) and a top wall (24) engaging a top shoulder of the needle support (10).

8. The drug delivery device according to any preceding claim wherein the spring (26) is in the form of a conical coil spring mounted between a top wall (24) of the needle shield cover (16) and a wall or support of a housing component of the delivery unit (3).

9. The drug delivery device according to any preceding claim, wherein the pumping system comprises a pump engine (38) including
- a stator (29),
- a rotor (31) rotatably and axially slidably mounted at least partially in the stator, the rotor comprising a first axial extension having a first diameter and the second axial extension having a second diameter greater than the first diameter,
- a first valve formed by a first valve seal mounted on the stator around the first axial extension, in conjunction with a first channel in the rotor that is configured to allow fluidic communication across the first valve seal when the first valve is in an open position, and
- a second valve formed by a second valve seal mounted on the stator around the second axial extension, in conjunction with a second channel in a rotor that is configured to allow fluidic communication across the second valve seal when the second valve is an open position.

10. The drug delivery device according to any preceding claim, wherein the needle actuation mechanism comprises an actuation disc (42) and an actuation lever (7), the actuation lever comprising a pivot portion (34) and a lever arm (36) extending therefrom configured to engage an indent (44) in the actuation disc (42) upon initial actuation of the drug delivery device.

11. The drug delivery device according to the preceding claim in conjunction with claim 8, wherein the actuation disc (42) is coupled to the rotor of the pump engine.

12. The drug delivery device according to claim 4 in conjunction with claim 10 wherein said pin (14b) extending from the needle support (10) is coupled to the lever arm (36).

13. The drug delivery device according to any preceding claim, further including a drive unit comprising an electronic control system, a power source, and a pump drive, wherein the pump drive comprises a rotary electrical motor and a coupling interface coupled to an output shaft of the rotary electrical motor, the coupling interface coupling to a drive coupling interface of the pumping system of the delivery unit, the pump drive providing torque to a rotor of the pumping system.
